# EUROPEAN PATENT SPECIFICATION

(11) **EP 0 357 155 B1**
(45) Date of publication and mention of the grant of the patent: **11.05.1994**
(21) Application number: 89202226.0
(22) Date of filing: 01.09.1989
(51) Int. Cl.: A61L 27/00, A61K 6/02

(54) **Prosthetic devices having bone-binding properties**
Prothesen mit knochenbindenden Eigenschaften
Articles de prothèse possédant la propiété de selier aux os

(30) Priority: 02.09.1988 NL 8802178
(43) Date of publication of application: 07.03.1990
(73) Proprietor: HC IMPLANTS B.V., NL-2333 CD Leiden (NL)
(72) Inventor: Bakker, Dirkjan, NL-2406 DD Alphen aan den Rijn (NL); Grote, Johannes Jacobus, NL-2381 NG Zoeterwoude (NL); Van Blitterswijk, Clemens Antoni, NL-2401 MD Alphen aand den Rijn (NL)
(74) Representative: de Bruijn, Leendert C.

(56) References cited:
- EP-A- 0 253 506
- DE-C- 3 644 588
- FR-A- 2 387 028
- FR-A- 2 476 480
- US-A- 4 350 806

## Description

The invention relates to prosthetic devices having bone-binding properties which are made of specific thermoplastic segmented copolymers which are compatible with biological materials. Further the invention relates to the therapeutic use of such prosthetic devices.

The ceramic "hydroxyapatite" is bioactive as concerns bonding to bone (C.A. van Blitterswijk et al., "Bioreactions at the tissue/hydroxyapatite interface", Biomaterials 6, pages 243-251 (1985). The so-called "lamina limitans"-like interface (LL-interface) at the interface between hydroxyapatite and bone which anorganic part mainly consists of hydroxyapatite, is characteristic for the chemical bond between both materials. More in particular said chemical bond is thought to be based on a bilateral crystal growth.

The sintered hydroxyapatite, however, belongs to the ceramics which are non-elastic materials. For that reason considerable research has been carried out for developing an elastic material having substantially the same chemical bone-bonding properties as hydroxyapatite.

FR-A-2,387,028 relates to prosthetic devices which are composed of an inner load bearing functional component and an outer porous coating of a biomedical thermoplast e.g. a thermoplastic polyester. The thermoplastic coating is provided in regions where long-term bone-fixation is desired by tissue ingrowth. For achieving above goal the materials of the thermoplastic coating must have the following properties:
(a) an average pore diameter of from about 90 to about 600 microns;
(b) pore interconnections having average diameters of greater than about 50 microns;
(c) a modulus of elasticity from about 1.72 10⁹ and about 2.07 10.¹⁰ Pa for the thermoplastic material;
(d) a porosity of greater than about 40%, for instance 40-70%, and
(e) a total creep strain of less than 1% at a constant stress of 6.9 10⁶ Pa at ambient temperature
and all the properties should be sufficient to enable stresses applied on the musculoskeletal system to be transferred to bone spicules within the pores of said material and maintain sufficient load and pore stability to promote irreversible ossification.

Surprisingly it has been found that prosthetic devices made of a segmented thermoplastic copolyester consisting essentially of a multiplicity of recurring long-chain ester units and short-chain ester units, said long-chain ester units comprising from 30-75% by weight of the copolyester and being represented by the formula

-OLO-CO-R-CO-

and said short-chain ester units being represented by the formula

-OEO-CO-R-CO-

where
- L: in said long-chain unit is a divalent radical remaining after removal of terminal hydroxyl groups from a poly(oxyethylene) glycol having a number average molecular weight of between 500 and 3,000 ;
- R: is a divalent radical remaining after removal of carboxyl groups from a dicarboxylic acid having a molecular weight of less than 300; and
- E: in said short-chain unit is an alkylene radical having 2-6 carbon atoms and optionally at least part of the surface of said device comprises macropores having a diameter in the range of 50-500 µm and a macroporosity of 10-90%,
are suitable bone-bonding implant materials.

More in particular the prosthetic devices according to the invention are made of thermoplastic copolymers wherein the poly(oxyethylene) glycol-units have a molecular weight of about 750-1,500 and in particular about 1,000. Further the symbol E is preferably a 1.2-ethylene or 1.4-butylene group i.e. the short-chain ester units are preferably based on either poly-1.2-ethylene terephthalate or poly-1.4-butylene terephthalate.

As deducible from the above the surface of the devices according to the invention may or may not comprise macropores. However, for an optimum fixation between said devices and bone at least a part of the bone-contacting surface thereof has macropores, preferably macropores having a diameter in the range of 60-250 µm and a macroporosity of 30-60%.

Due to the fact that the copolymers according to the invention do have neither a crystal stoechiometry nor a crystal structure which corresponds to the crystal stoechiometry or crystal structure of hydroxyapatite it is brought to the fore that the bone-bonding process will have a different mechanism compared to the hydroxyapatite-bone-bonding process.

The segmented copolymers which are used in the prosthetic devices according to the invention are more or less known from the prior art. For instance in the examples of U.S. patent 3,908,201 copolymers of poly(ethyleneglycol) and bis(beta-hydroxyethyl) terephthalate are disclosed. The average molecular weight of the preferably used poly(ethyleneglycol)-units was about 4,000. Said molecular weight of 4,000 corresponds to the preferred value for the CH₂-CH₂-O-units as mentioned in column 7, line 2 of said U.S. patent. Further U.S. patent 3,908,201 discloses a copolymer containing 50% by weight of units derived from poly(ethyleneglycol) of average molecular weight of 600 and 50% by weight of units derived from poly(ethylene terephthalate).

Hydantoin-containing segmented copolymers are known from U.S. patent 4,350,806. The reason for the presence of the hydantoin radical in the polyoxyethylene-unit was lain in the reduction of the polyoxyethylene polymer's propensity to crystallize and further provides a chemically reactive side for incorporating other moieties to enhance certain properties. On account of this "foreign" radical it was possible to substantially increase the molecular weight of the polyether-unit.

In view of the contents of said U.S. patents 3,908,201 and 4,350,806 it is emphasized that no indication whatsoever is given concerning the condition that the average molecular weight of the polyoxyethylene unit should lie within the range of 500-3,000, preferably in the range of 750-1,500 and most preferably should amount about 1,000 for achieving bone-bonding properties. Further nothing is mentioned about the presence of macropores.

On account of the fact that several useful copolyesters as well as preparation methods therefore are described in U.S. patents 3,908,201, 4,262,114 and 4,350,806 said three references are hereby incorporated in its entirety by reference.

From investigation of Applicant concerning polyoxyethylene/polybutyleneterephthalate-copolymers (PEO/PBT-copolymers; prepared according to the process indicated in Example 2) it appeared that middle ear epithelium cells of a rat attached themselves and proliferated on films (thickness: 100 µm) of the following copolymers:
1) 30 wt.% PEO / 70 wt.% PBT
2) 40 wt.% PEO / 60 wt.% PBT
3) 55 wt.% PEO / 45 wt.% PBT
4) 60 wt.% PEO / 40 wt.% PBT
5) 70 wt.% PEO / 30 wt.% PBT.

The above epithelium cells cultured on said films for 7 and 12 days had the same morphology as the cells cultured on tissue culture polystyrene. The best growth results of the epithelium cells in question were achieved with the 40/60- and 55/45 - PEO/PBT-films.

Above investigation was carried out with a culture of middle ear epithelium cells of a rat cultured according to the reference: Van Blitterswijk et al., Culture and Characterization of Rat Middle-ear Epithelium, Acta Otolaryngol (Stockholm) 101 (1986), pages 453-466.

The prosthetic devices according to the invention may be used on several medical areas like
a) surgery in the head-neck area; for instance as (1) an artificial tympanic membrane for instance in the total middle ear prosthesis, (2) a covering of the middle ear cavity, (3) artificial ossicles, (4) an artificial palate and (5) a ventilation tube;
b) plastic surgery and maxillofacial surgery; for instance for bone augmentation with respect to the nose, chin, cheekbone and eye socket;
c) jaw surgery and dentistry; for instance as a filling material for extraction cavities and as a dental root implant; and
d) orthopedics; for instance as a bone-replacing or cartilage-replacing material and for fracture fixation.

The prosthetic devices according to the invention may also be combined with other materials, for instance as a composite material with a.o. hydroxyapatite or polylactic acid as a coating or as a blend with the instance polylactic acid.

Further the shape of the prosthetic devices according to the invention may vary considerably, dependent on the application. Examples of shapes are films, plates, screws and filaments, for instance in the form of sutures.

The invention is elucidated with the following examples which should not be interpreted in a restricted way.

### Example 1

The bone-bonding strengths of PEO/PBT (55/45 w/w) comprising poly(ethyleneglycol)-units having an average molecular weight of about 1000 (prepared according to Example 3 of U.S. patent 4,262,114 with the proviso that PEO was used instead of HPEO and the amount of Ionox 330 was 0.5 weight %) are compared with the bone-bonding strengths of hydroxyapatite and tetracalcium phosphate acting as positive controls and with the bonebonding strength of silicone rubber acting as a negative control. For the purpose of this experiment implants of 2x2x1.5 mm made of the three above indicated materials were implanted in the tibia of male Wistar-rats (body weight: 300 g).

Three weeks after the implantation the hydroxyapatite implants and the tetracalcium phosphate implants were bound to the bone in such a way that a "push-out"-pressure of about 1 MPa (measured by means of a thermomechanic analysis device (Mettler)) was not sufficient for removing the implants from the implantation bed. The silicone rubber implants were surrounded by an envelope of fibrous tissue and came already loose during the preparation of the sample. The bone-bonding strength of the silicone rubber implant was less than about 0.01 MPa. With respect to the PEO/PBT-implants it is reported that said implants were bound to bone. The bone-bonding strength of both copolymers was in the range of 1 MPa.

Six weeks and twenty six weeks respectively after the implantation the PEO/PBT-implants were bound to the bone with a bonding strength of about 4 MPa. In this respect it is remarked that not the bonding strength was the limitative factor but the strength of the polymer itself. All implants made of PEO/PBT fractured before that they could be pushed out of the tibia. For the sake of completeness it is reported that the implants made of hydroxyapatite and tetracalcium phosphate respectively tolerated a "push-out"-pressure of about 7 MPa; at a higher pressure said implants also fractured.

From this example it is clear that pore-free implants made of PEO/PBT are also chemically bound to bone, i.e. the contact zone of the copolymers in question with the bone was characterized by an electron-dense structure, the so-called "lamina limitans"-like interface.

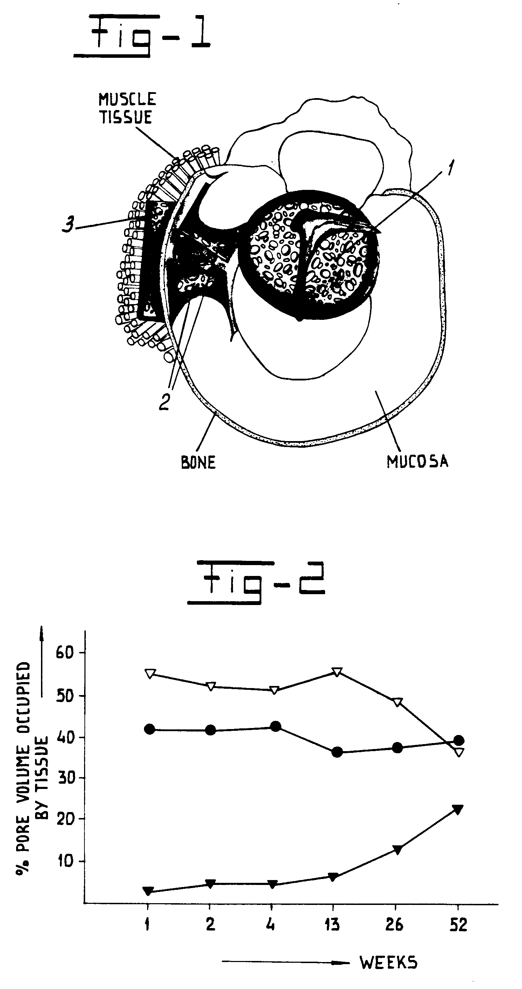

## Claims

1. A prosthetic device having bone-bonding properties and made of a segmented thermoplastic copolyester consisting essentially of a multiplicity of recurring long-chain ester units and short-chain ester units, said long-chain ester units comprising from 30-75% by weight of the copolyester and being represented by the formula
-OLO-CO-R-CO-
and said short-chain ester units being represented by the formula
-OEO-CO-R-CO-
where
L in said long-chain unit is a divalent radical remaining after removal of terminal hydroxyl groups from a poly(oxyethylene) glycol having a number average molecular weight of between 500 and 3,000 ;
R is a divalent radical remaining after removal of carboxyl groups from a dicarboxylic acid having a molecular weight of less than 300; and
E in said short-chain unit is an alkylene radical having 2-6 carbon atoms and
optionally at least part of the surface of said device comprises macropores having a diameter in the range of about 50-500 µm and a macroporosity in the range of 10-90%.

2. The prosthetic device of Claim 1, made of a thermoplastic copolyester, wherein said poly(oxyethylene) glycol-unit L has an average molecular weight of 750 - 1,500.

3. The prosthetic device of Claim 1 made of a thermoplastic copolyester, wherein said poly(oxyethylene) glycol-unit L has an average molecular weight of about 1,000.

4. The prosthetic device of Claim 1 made of a thermoplastic copolyester, wherein substantially all of said dicarboxylic acid is terephthalic acid.

5. The prosthetic device of Claim 1 made of a thermoplastic copolyester, wherein said alkylene group having 2-6 carbon atoms is a C_{1.2}-ethylene or C_{1.4}-butylene group.

6. A prosthetic device according to Claim 1, where at least a part of the surface of the device comprises macropores having a diameter in the range of 60-250 µm and a macroporosity of 30-60%.

## Patentansprüche

1. Prothetische Einrichtung mit knochenbindenden Eigenschaften und hergestellt aus einem im wesentlichen aus einer Vielzahl von sich wiederkehrenden, langkettigen Estereinheiten und kurzkettigen Estereinheiten bestehenden segmentierten, thermoplastischen Copolyester, wobei die langkettigen Estereinheiten von 30 bis 75 Gew.-% des Copolyesters aufweisen und durch die nachfolgende Formel
-OLO-CO-R-CO-
und die kurzkettigen Estereinheiten durch die nachfolgende Formel
-OEO-CO-R-CO-
dargestellt werden, in denen
L in den langkettigen Einheiten ein zweiwertiger Rest ist, der nach Entfernung der endständigen Hydroxylgruppen aus einem Poly(oxyethylen)glycol mit einem durchschnittlichen Molekulargewicht von zwischen 500 und 3000 verbleibt,
R ein zweiwertiger Rest ist, der nach Entfernung der Carboxylgruppen aus einer Dicarbonsäure mit einem Molekulargewicht von weniger als 300 verbleibt und
E in den kurzkettigen Einheiten ein Alkylenrest mit 2 bis 6 Kohlenstoffatomen ist und
gegebenenfalls zumindest ein Teil der Oberfläche der Einrichtung Makroporen mit einem
Durchmesser im Bereich von etwa 50 bis 500 µm und einer Makroporosität im Bereich von 10 bis 90 % aufweist.

2. Aus einem thermoplastischen Copolyester hergestellte prothetische Einrichtung nach Anspruch 1, worin die Poly(oxyethylen)glycol-Einheit L ein durchschnittliches Molekulargewicht von 750 bis 1500 hat.

3. Aus einem thermoplastischen Copolyester hergestellte prothetische Einrichtung nach Anspruch 1, worin die Poly(oxyethylen)glycol-Einheit L ein durchschnittliches Molekulargewicht von etwa 1000 hat.

4. Aus einem thermoplastischen Copolyester hergestellte prothetische Einrichtung nach Anspruch 1, worin im wesentlichen die gesamte Dicarbonsäure Terephthalsäure ist.

5. Aus einem thermoplastischen Copolyester hergestellte prothetische Einrichtung nach Anspruch 1, worin die Alkylengruppe mit 2 bis 6 Kohlenstoffatomen eine C_{1,2}-Ethylen- oder C_{1,4}-Butylen-Gruppe ist.

6. Prothetische Einrichtung nach Anspruch 1, in der zumindest ein Teil der Oberfläche der Vorrichtung Makroporen mit einem Durchmesser im Bereich von 60 bis 250 µm und eine Makroporosität von 30 bis 60 % aufweist.

## Revendications

1. Dispositif prothétique présentant des propriétés de fixation aux os et réalisé en un copolyester thermoplastique segmenté constitué essentiellement d'une multiplicité de motifs répétitifs esters à longue chaîne et esters à courte chaîne, les motifs esters à longue chaîne constituant de 30 à 75 % en poids du copolyester et étant représentés par la formule suivante :
-OLO-CO-R-CO-
et les motifs esters à courte chaîne étant représentés par la formule suivante :
-OEO-CO-R-CO-
où
L dans le motif à longue chaîne est un radical divalent restant après enlèvement des groupes hydroxyles terminaux d'un poly(oxyéthylène)glycol ayant une masse moléculaire moyenne en nombre de 500 à 3.000 ;
R est un radical divalent restant après enlèvement des groupes carboxyles d'un acide dicarboxylique ayant une masse moléculaire inférieure à 300 ; et
E dans le motif à courte chaîne est un radical alkylène ayant de 2 à 6 atomes de carbone, et éventuellement, au moins une partie de la surface du dispositif comprend des macropores ayant un diamètre d'environ 50 à 500 µm et une macroporosité de 10 à 90 %.

2. Dispositif prothétique selon la revendication 1, réalisé en un copolyester thermoplastique, dans lequel le motif poly(oxyéthylène)glycol L a une masse moléculaire moyenne de 750 à 1.500.

3. Dispositif prothétique selon la revendication 1, réalisé en un copolyester thermoplastique, dans lequel le motif poly(oxyéthylène)glycol L a une masse moléculaire moyenne d'environ 1.000.

4. Dispositif prothétique selon la revendication 1, réalisé en un copolyester thermoplastique, dans lequel la presque totalité de l'acide dicarboxylique est l'acide téréphtalique.

5. Dispositif prothétique selon la revendication 1, réalisé en un copolyester thermoplastique, dans lequel le groupe alkylène ayant de 2 à 6 atomes de carbone est un groupe éthylène en C_{1,2} ou un groupe butylène en C_{1,4}.

6. Dispositif prothétique selon la revendication 1, dans lequel au moins une partie de la surface du dispositif comprend des macropores ayant un diamètre de 60 à 250 µm et une macroporosité de 30 à 60 %.
